# EUROPEAN PATENT SPECIFICATION

(11) **EP 1 911 417 B1**
(45) Date of publication and mention of the grant of the patent: **24.04.2013**
(21) Application number: 06761594.8
(22) Date of filing: 27.07.2006
(51) Int. Cl.: A61F 2/06, A61L 27/22, A61L 27/36, A61L 33/12

(54) **BIOLOGICAL ARTIFICIAL BLOOD VESSEL AND PREPARATION METHOD THEREOF**
BIOLOGISCHES KÜNSTLICHES BLUTGEFÄSS UND HERSTELLUNGSVERFAHREN
VAISSEAU SANGUIN ARTIFICIEL BIOLOGIQUE ET SON PROCEDE DE PREPARATION

(30) Priority: 29.07.2005 CN 200510036175
(43) Date of publication of application: 16.04.2008
(73) Proprietor: Summit (GD) Biotech Co., Ltd., Uandong 510663 (CN)
(72) Inventor: XU, Guofeng, Guangzhou, Guangdong 510630 (CN)
(74) Representative: Korga, Leokadia
(86) International application number: PCT/CN2006/001880
(87) International publication number: WO 2007/012282

(56) References cited:
- CN-A- 1 237 889
- CN-A- 1 313 741
- CN-A- 1 445 003
- US-A- 4 083 066
- US-A- 4 319 363
- US-A1- 2001 044 654
- MATTILA S P ET AL: "Antigenicity of vascular heterografts" JOURNAL OF SURGICAL RESEARCH 1973, vol. 15, no. 2, 1973, pages 81-86, XP002490366 ISSN: 0022-4804
- TOMIZAWA: 'NLM3143386 Development of a small-caliber vascular graft with antithrombogenicity induced by extreme hydrophilicity.' ASAIO TRANSACTIONS / AMERICAN SOCIETY FOR ARTIFICIAL INTERNAL ORGANS 01 January 1988, XP055044341

## Description

The present invention relates to a method for preparation of a medical prosthesis for human implantation, and in particular, to an artificial blood vessel which is utilized to replace damaged blood vessels, or as a device for bypassing blood vessel.

Vascular diseases have become one of the most widespread diseases that threaten human health and life. One of the methods for treating vascular diseases is to use artificial blood vessels to replace the diseased blood vessel, or to bypass the diseased portion of the vessel. Currently, the most commonly-used artificial blood vessels for clinical application are knitted Dacron tubes or expanded polytetrafluoroethylene tubes, both being produced from synthetic materials which can be used to form internal pseudomembranes and to maintain long-term smooth passage of blood. Unfortunately, all foreign matters that are implanted into a human body face some degree of chronic rejection sooner or later leading to an adverse reaction in the body. In addition, the anticoagulation of these conventional artificial blood vessels is poor, and usually only artificial blood vessels having a diameter of at least a 6 mm provide good passage, so that smaller-diameter artificial blood vessels, especially those having diameters of less than 4 mm, often result in embolisms after being implanted.

There have been many studies using animal blood vessels as artificial blood vessels for humans, but none have become viable for clinical application because the techniques for treatment are outdated and lacking. For example, conventional treatment methods include fixing an animal blood vessel with glutaraldehyde, followed by defatting and cell removal, and then the product is directly implanted. Treatment with glutaraldehyde is for fixing the protein molecules in the animal tissue through crosslinking by the acetal reaction, but toxic glutaraldehyde is slowly released due to degradation after the animal blood vessel treated in this manner is implanted into a human body, thereby inhibiting the production of endothelial cells in the blood vessel. Migneault et al, BioTechniques, 2004, 37, 790-802). In addition, conventional treatment methods employ cell removal as an effective means for eliminating or removing antigens US 2001/0044654, but according to research results in molecular biology and molecular immunology, antigenicity does not only originate from the cell, but also from active groups at certain specific locations on the proteins and polysaccharides, or specific conformations. These specific groups or conformations are called antigenic determinants clusters, and antigens can only be eliminated by blocking the active groups of the antigenic determinants and altering the specific conformation of the antigenic determinants; antigens cannot be effectively eliminated by cell removal.

Accordingly, the conventional methods for treating animal blood vessels do not totally eradicate chronic immune rejection due to the toxic presence of residual glutaraldehyde and the incomplete elimination of the antigens, making it very difficult for the endothelial cells and other vascular cells of the host blood vessels to migrate into and grow in the artificial blood vessel, so that the expected effects cannot be attained.

It is an object of the present invention to provide a biological artificial blood vessel that overcomes the disadvantages described above, while having good biocompatibility, minimal residual toxicity, and minimal chronic delayed immune rejection.

It is another object of the present invention to provide a method of preparing a biological artificial blood vessel that overcomes the disadvantages described above, while having good biocompatibility, minimal residual toxicity and minimal chronic delayed immune rejection

The biological artificial blood mentioned in this present invention is composed of a substrate made of an animal blood vessel, and an active coating bonding to the inner surface of the substrate. The animal blood vessel is immobilized by crosslinking with an immobilizing agent and is treated to remove antigens. The active coating contains anti-coagulation components.

Animal blood vessel tissues comprise mainly collagens and glucosaminoglycans which are easily degraded or decomposed by microorganisms. Conventionally, aldehydes (formaldehyde, glutaraldehyde, etc.) are utilized for crosslinking and fixation to increase their stability. However, aldehydes undergo crosslinking with proteins through aldolization, and toxic aldehydes are released when the crosslinked products are degraded, so that products fixed with an aldehyde have long-term residual toxicity. When epoxides, diamides, diisocyanates or carbodiimides are utilized as fixatives in place of aldehydes, this toxicity problem can be eliminated. When an epoxide is utilized, for example, proteins are crosslinked through the ring opening reaction of the epoxide, and reverse ring closure to form the epoxide back does not readily occur, and the degradation products are polyols which can be metabolized by the body so that there is no risk of toxic aldehyde radicals. The stability of the animal blood vessels after treatment is also higher than those fixed with aldehydes. According to modern immunological theory, the antigenicity of animal tissues stems mainly from active groups located at specific sites and in specific conformations, and these active groups include -OH, -NH₂, -SH, etc. The specific conformations result mainly from specific hydrogen bonds formed by spiral protein chains. The specific sites and conformations are called antigenic determinants. When treating animal blood vessels, one or several small, active reagents (e.g., acid anhydrides, acetyl chlorides, acrylamides, epoxides, etc.), which can readily react with these groups, are used to bind and block these groups, which in turn effectively minimizes the antigenicity, and in the meantime strong hydrogen bonding reagents (e.g., guanidine compounds) are utilized to form new hydrogen bonds and replace the inherent hydrogen bonding of the specific conformations. This changes the specific conformations and further effectively minimizes the antigenicity.

The tissues of the animal vessels cannot be easily altered after they have been crosslinked and fixed by epoxides, thereby leaving no residual toxicity. The immunogenicity is effectively minimized by blocking the active groups in the proteins and changing the conformation, and the formed substrate has high stability, while exhibiting no chronic immune rejection and has excellent biocompatibility. Furthermore, containing anti-coagulation components, the active coating binding by covalent bond to the inner surface of the substrate cannot be flushed away, so as to keep long-term anticoagulation effect to ensure the long-term blood flow after transplantation.

The anti-coagulant agents are substances carrying a negative charge on the surface layer, but an excessively strong negative charge on the surface is detrimental to the growth and proliferation in the blood vessels of the endothelial cells (which also carry a negative charge). For this reason, heparin is the preferred anti-coagulant.

As a optimal strategy, the coating also contains specific polypeptides to adhere to growth factors, which have broad-spectrum adhesion and accumulation of blood growth factors, such as vessel endothelial growth factor (VEGF), fibroblast growth factor (FGF), platelet-derived growth factor (PDGF-bb), vessel permeation factor (VPF), so as to promote formation of blood vessels. One of the polypeptides consist of 16 lysines (K16), glycine (G), arginine (R), aspartic acid (D), serine (S), proline (P) and cysteine (C), and its sequence is K16-G-R-G-D-S-P-C.

In certain embodiments, the substrate has the shape of a straight tube, a U-shape tube, or a C-shape tube, or a Y-shape tube, in order to adapt to various transplanting sites.

With natural animal blood vessels as the substrate, the preparation method for the biological artificial blood comprises the following steps:
1) Pretreatment: Initial sterilization is performed using a broad spectrum, highly-effective, low-toxicity bactericide, followed by trimming irregular portions.
2) Defatting: The fatty substances in the substrate are extracted with organic solvents.
3) Fixation: The protein molecules in the substrate are crosslinked and fixed with a fixative as defined in [0015],
4) Antigen elimination: The specific active groups of proteins in the substrate, such as -OH, -NH₂, -SH, and so on, are blocked with active reagents, and the specific conformation of them is changed, by substituting reagents with strong hydrogen bonds for the special hydrogen bonds in helix chains of protein molecules in the substrate.
5) Anti-coagulation modification: The anti-coagulation components are bonded to the inner surface of the substrate with a coupling agent, finally forming the coating with high anti-coagulation activity.

As an optimal strategy, in the method for preparation biological artificial blood, the surface of a coating is formed by coupling with a coupling agent as to bind a polypeptide, which has broad-spectrum adhesion and accumulation of blood growth factors.

In the preparation method of biological artificial blood, the epoxy compound used as a fixative is a monoepoxy compound of the following formula: 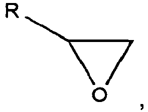 a diepoxy compound of the following formula 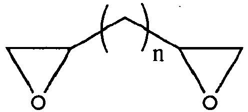 an oligoepoxy compound, such as polypropylene oxide; and R = -CₙH₂ₙ₊₁, andn=0-10.

In the preparation method of biological artificial blood, the active reagents are organic acid anhydrides with low molecular weight, acetyl chlorides, acrylamides or monocyclic oxides, and the reagents forming strong hydrogen bonds are guanidine compounds.

The anti-coagulation components described above are substances which make the coating load negative charges, and are also heparin with high activity of anticoagulation. The coupling agent is an epoxide, anhydride, or diacyl chloride, and so on.

In the preparation method of biological artificial blood, the coupling agent used to couple with the polypeptide component is diacryl diamide, diepoxidate, or other reagent having double functional groups which undergo aldolization with -NH₂, -OH, and -COOH.

The advantages of this invention lie in the follwoing: (a) due to complete elimination of antigens, the treated animal blood vessel as a substrate has a high stability, excellent biocompatibility with the blood in the host so as to prevent coagulation and ensure long-term blood flow after being transplanted into the human body; (b) because basic components are similar to the human body, and the final degraded products of 20 amino acids and sugar, it may be absorbed by tissues in the human body, and has excellent histocompatability; (c) it is a good carrier to stimulate regeneration of blood vessels, which can induce growth into it of tissues of the blood vessel; (d) after coupling the polypeptide, it can also accumulate growth factors to promote growth of the endolethial cells and formation of the new blood vessels, and finally be assimilated into newly-generated tissues of the blood vessel; and (e) its performance is significantly better that that of chemically synthesized materials used for prosthesis, so that it is especially suitable for making those endoprosthesises with a diameter of less than 6mm.

FIG. 1 is a perspective view of an artificial blood vessel according to one embodiment of the present invention.

FIG. 2 is a cross-sectional view of the artificial blood vessel of FIG. 1.

FIG. 3 is a perspective view of an artificial blood vessel according to another embodiment of the present invention.

FIG. 4 is a perspective view of an artificial blood vessel according to another embodiment of the present invention.

FIG. 5 is a perspective view of an artificial blood vessel according to another embodiment of the present invention.

1. Substrate; 2 Coating

### EXAMPLES

Example 1

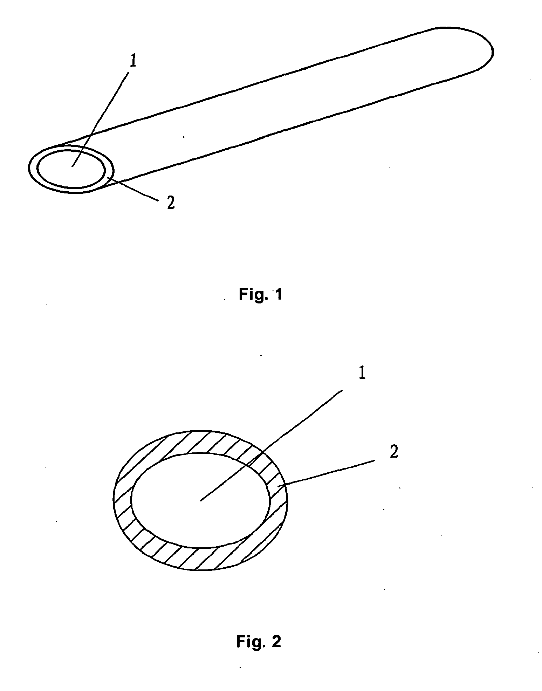

Referring to FIGS. 1 and 2, the biological artificial blood vessel according to the present invention comprises a substrate 1 comprising a natural animal blood vessel that has been crosslinked and fixed with an epoxide, treated to minimize antigens, and a coating 2 bound to the inner surface of substrate 1 and having an anticoagulant component. The substrate can be a straight tube and the anticoagulant agent in the coating 2 is heparin. The coating 2 also contains a polypeptide consisting of 16 lysines (K16), glycine (G), arginine (R), aspartic acid (D), serine (S), proline (P) and cysteine (C).

This biological artificial vessel was prepared in the following steps:
1. Pretreatment: Initial sterilization was performed using a broad spectrum, highly-effective, low-toxicity bactericide such as benzalkonium chloride or chlorhexidine, followed by trimming excess tissue.
2. Defatting: The fatty substances in the substrate 1 were extracted with an organic solvent such as chloroform, ethyl acetate, anhydrous alcohol or mixtures thereof.
3. Fixation: The protein in the substrate 1 was crosslinked and fixed with a diepoxide 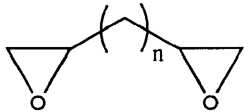 , where n=0-10.
4. Antigen elimination: An active reagent with a low molecular weight such as organic acid anhydride, acyl chloride, acylamide or monocyclic oxide was utilized to block the specific active groups such as -OH, -NH₂, -SH, etc., in the proteins of the substrate 1, and a reagent having strong hydrogen bond, such as a guanidine compound, was utilized to replace the specific hydrogen bonding on the spiral chains of the protein molecules in the substrate and alter its specific conformation.
5. Anticoagulant modification: A coupling agent was utilized to couple the anticoagulant (heparin) to the inner surface of the substrate to form the active coating with high activity of anti-coagulation.
6. Coupling polypeptide: A diamide was utilized as a coupling agent to couple a polypeptide consisting of 16 lysines (K16), glycine (G), arginine (R), aspartic acid (D), serine (S), proline (P) and cysteine (C) capable of binding a wide variety of growth factors on surface layer 2, and the sequence of the composition of the polypeptide is K16-G-R-G-D-S-P-C.

Example 2

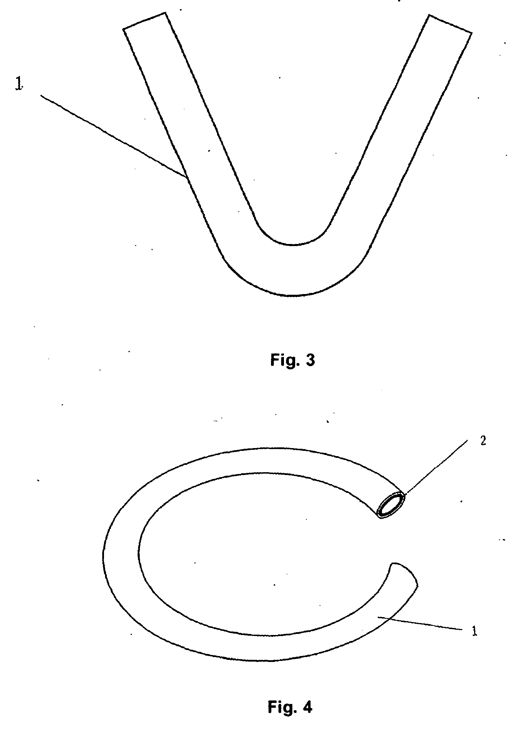

Referring to FIG. 3, the substrate 1 is a U-shaped tube and a diisocyanate was utilized for fixing the protein molecules while all the other technical characteristics are the same as those of Example 1.

Example 3

Referring to FIG. 4, the substrate 1 is a C-shaped tube and a diamide was utilized for fixing the protein molecules while all other technical characteristics are the same as those of Example 1.

Example 4

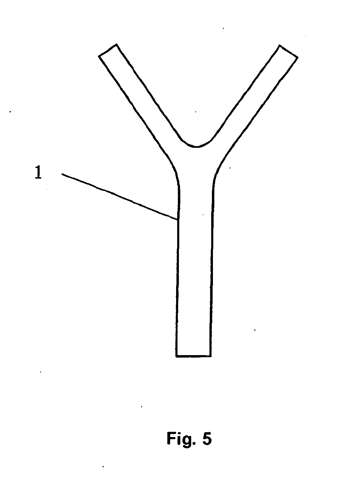

Referring to FIG. 5, the substrate 1 is a Y-shaped tube while all other technical characteristics are the same as those of Example 1.

## Claims

1. A method for preparing a biological artificial blood vessel, using natural animal blood vessels as the substrate 1, comprising the following steps:
1) Pretreatment: Initial sterilization is performed using a broad spectrum, highly-effective, low-toxicity bactericide, followed by trimming irregular portions;
2) Defatting: The fatty substances in the substrate are extracted with organic solvents;
3) Fixation: The protein molecules in the substrate are crosslinked and fixed with a fixative, said fixative being a monoepoxy compound of the following formula: 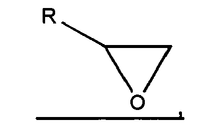 a diepoxy compound of the following formula 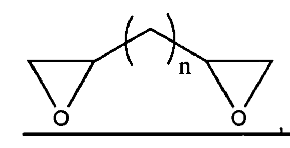 or
an oligoepoxy compound, such as polypropylene oxide; and R = -CₙH₂ₙ₊₁, and n=0-10;
4) Antigen elimination: The specific active groups of proteins in the substrate, such as -OH, -NH₂, -SH, and so on, are blocked with active reagents, and the specific conformation of them is changed, by substituting reagents with strong hydrogen bonds for the special hydrogen bonds in helix chains of protein molecules in the substrate; and
5) Anti-coagulation modification: The anti-coagulation components are bonded to the inner surface of the substrate 1 with a coupling agent, finally forming the coating 2 with high activity of anti-coagulation.

2. The method for preparing a biological artificial blood vessel of claim 1, wherein said coating 2 is formed by coupling with a coupling agent so as to bind a polypeptide, which has broad-spectrum adhesion and accumulation of blood growth factors.

3. The method for preparing a biological artificial blood vessel of claim 2, wherein said polypeptide consists of 16 lysines (K16), glycine (G), arginine (R), aspartic acid (D), serine (S), proline (P) and cysteine (C), and sequence of the polypeptide is K16-G-R-G-D-S-P-C.

4. The method for preparing a biological artificial blood vessel of claim 3, wherein said active reagents are organic acid anhydrides with low molecular weight, acetyl chlorides, acrylamides or monocyclic oxides, and the reagents forming strong hydrogen bonds are guanidine compounds.

5. The method for preparing a biological artificial blood vessel of claim 1, wherein the coupling agent is an epoxy compound, an anhydride compound, or a diacyl chloride compound.

6. The method for preparing a biological artificial blood vessel of claim 2, wherein the coupling agent is an acetyl diamide compound, a dioic anhydride compound, a diepoxy compound, or any other compounds with double functional groups, which participates in a condensation reaction with groups, such as -NH₂, -OH, or -COOH.

7. The method for preparing a biological artificial blood vessel of claim 1, wherein the substrate 1 has been crosslinked, and antigens on the substrate 1 have been minimized, and the substrate 1 has an inner surface to which an anticoagulant is coupled to form the coating 2.

8. The method for preparing a biological artificial blood vessel of claim 2, wherein said coating 2 components are substances which make the coating load negative charges, or heparins with high anticoagulative activity.

9. The method for preparing a biological artificial blood vessel of claim 2, wherein one of said polypeptide consists of 16 lysines (K16), glycine (G), arginine (R), aspartic acid (D), serine (S), proline (P) and cysteine (C).

10. The method for preparing a biological artificial blood vessel of claim 9, wherein the said substrate 1 has the shape of a straight tube, a U-shaped tube, or a C-shaped tube, or a Y-shaped tube.

## Patentansprüche

1. Verfahren zur Herstellung eines biologischen künstlichen Blutgefäßes unter Verwendung von natürlichen tierischen Blutgefäßen als Trägerschicht 1, das die folgenden Schritte umfasst:
1) Vorbehandlung: Durchführung einer Anfangssterilisation unter Verwendung eines hochwirksamen, Breitspektrumbakterizid von niedriger Toxizität gefolgt von einem Zuschneiden in unregelmäßige Abschnitte;
2) Entfettung: Extraktion von Fettsubstanzen in der Trägerschicht mit organischen Lösemitteln;
3) Fixierung: Quervernetzung und Fixierung der Proteinmoleküle in der Trägerschicht mit einem Fixiermittel, wobei das Fixiermittel eine Monoepoxyverbindung der folgenden Formel ist: 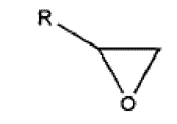 eine Diepoxyverbindung der folgenden Formel: 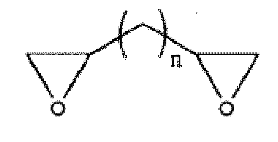 oder
eine Oligoepoxyverbindung, wie etwa Polypropylenoxid; wobei R = -CₙH₂ₙ₊₁ und n = 0-10;
4) Antigenentfernung: Spezifische aktive Gruppen der Proteine in der Trägerschicht, wie etwa -OH, -NH₂, -SH und so weiter, werden mit aktiven Reagenzien blockiert und deren spezifische Konformation wird verändert durch Substituierung von Reagenzien mit starken Wasserstoffbindungen für die spezifischen Wasserstoffbindungen in Helixketten der Proteinmoleküle der Trägerschicht; und
5) Antikoagulationsänderung: Die Antikoagulationsverbindungen werden an die innere Oberfläche der Trägerschicht 1 mit einem Koppelungsmittel gebunden, was schließlich zur Ausbildung der Schicht 2 mit einer hohen Antikoagulationsaktivität führt.

2. Verfahren zur Herstellung eines biologischen künstlichen Blutgefäßes nach Anspruch 1, wobei die Schicht 2 durch Koppeln mit einem Koppelungsmittel gebildet wird, so dass ein Polypeptid gebunden wird, das eine Breitspektrumadhäsion und -akkumulation von Blutwachstumsfaktoren aufweist.

3. Verfahren zur Herstellung eines biologischen künstlichen Blutgefäßes nach Anspruch 2, wobei das Polypeptid aus 16 Lysinen (K16), Glycin (G), Arginin (R), Asparaginsäure (D), Serin (S), Prolin (P) und Cystein (C) besteht und die Sequenz des Polypeptids K16-G-R-G-D-S-P-C ist.

4. Verfahren zur Herstellung eines biologischen künstlichen Blutgefäßes nach Anspruch 3, wobei die reaktiven Reagenzien Anhydride organischer Säuren mit einem niedrigen Molekulargewicht, Acetylchloride, Acrylamide oder monozyklische Oxide sind und die Reagenzien, welche starke Wasserstoffbindungen ausbilden, Guanidinverbindungen sind.

5. Verfahren zur Herstellung eines biologischen künstlichen Blutgefäßes nach Anspruch 1, wobei das Koppelungsmittel eine Epoxyverbindung, eine Anhydridverbindung oder eine Diacylchloridverbindung ist.

6. Verfahren zur Herstellung eines biologischen künstlichen Blutgefäßes nach Anspruch 2, wobei das Koppelungsmittel eine Acetyldiamidverbindung, eine Dioicanhydridverbindung, eine Diepoxyverbindung oder eine beliebige andere Verbindung mit doppelfunktionellen Gruppen ist, die an einer Kondensationsreaktion mit Gruppen wie etwa -NH₂, -OH oder -COOH beteiligt ist.

7. Verfahren zur Herstellung eines biologischen künstlichen Blutgefäßes nach Anspruch 1, wobei die Trägerschicht 1 quervernetzt wurde und Antigene auf der Trägerschicht 1 minimiert wurden und die Trägerschicht 1 eine innere Oberfläche aufweist, an die ein Antikoagulans gekoppelt ist, so dass Schicht 2 gebildet wird.

8. Verfahren zur Herstellung eines biologischen künstlichen Blutgefäßes nach Anspruch 2, wobei die Bestandteile der Schicht 2 Substanzen sind, welche die Schicht zum Aufnehmen von negativen Ladungen veranlassen oder Heparine mit hoher antikoagulativer Wirkung.

9. Verfahren zur Herstellung eines biologischen künstlichen Blutgefäßes nach Anspruch 2, wobei eines der Polypeptide aus 16 Lysinen (K16), Glycin (G), Arginin (R), Asparaginsäure (D), Serin (S), Prolin (P) und Cystein (C) besteht.

10. Verfahren zur Herstellung eines biologischen künstlichen Blutgefäßes nach Anspruch 9, wobei die Trägerschicht 1 die Gestalt einer geraden Röhre, einer U-förmigen Röhre, einer C-förmigen Röhre oder einer Y-förmigen Röhre annimmt.

## Revendications

1. Procédé de préparation d'un vaisseau sanguin artificiel biologique, en utilisant des vaisseaux sanguins naturels d'animaux en tant que substrat 1, ledit procédé comprenant les étapes suivantes :
1) prétraitement : la stérilisation initiale est réalisée en utilisant un bactéricide à large spectre, très efficace et à faible toxicité, suivie par la découpe des parties irrégulières ;
2) dégraissage : les substances grasses dans le substrat sont extraites avec des solvants organiques ;
3) fixation : les molécules protéiques dans le substrat sont réticulées et fixées avec un fixateur, ledit fixateur étant un composé monoépoxy répondant à la formule suivante : 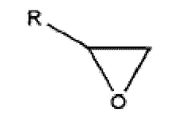 un composé diépoxy répondant à la formule suivante : 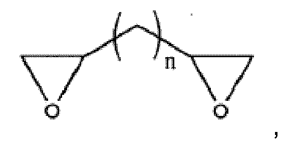 ou
un composé oligoépoxy, tel que l'oxyde de polypropylène ; et
R = -CₙH₂ₙ₊₁, et n = 0 à 10 ;
4) élimination des antigènes : les groupes actifs spécifiques de protéines dans le substrat, tels que -OH, -NH₂, -SH, et ainsi de suite, sont bloqués avec des réactifs actifs, et leur conformation spécifique est modifiée, par substitution de réactifs avec des liaisons hydrogène fortes aux liaisons hydrogène spéciales dans les chaînes hélicoïdales de molécules protéiques dans le substrat ; et
5) modification anticoagulation : les composants anticoagulation sont liés à la surface interne du substrat 1 avec un agent de couplage, formant finalement le revêtement 2 avec une grande activité anticoagulation.

2. Procédé de préparation d'un vaisseau sanguin artificiel biologique selon la revendication 1, dans lequel ledit revêtement 2 est formé par couplage avec un agent de couplage de manière à lier un polypeptide, qui présente une adhésion de large spectre et une accumulation de facteurs de croissance sanguins.

3. Procédé de préparation d'un vaisseau sanguin artificiel biologique selon la revendication 2, dans lequel ledit polypeptide est constitué de 16 lysines (K16), glycine (G), arginine (R), acide aspartique (D), sérine (S), proline(P) et cystéine (C), et la séquence du polypeptide est K16-G-R-G-D-S-P-C.

4. Procédé de préparation d'un vaisseau sanguin artificiel biologique selon la revendication 3, dans lequel lesdits réactifs actifs sont des anhydrides d'acides organiques ayant une faible masse moléculaire, des chlorures d'acétyle, des acrylamides ou des oxydes monocycliques, et les réactifs formant des liaisons hydrogène fortes sont des composés guanidine.

5. Procédé de préparation d'un vaisseau sanguin artificiel biologique selon la revendication 1, dans lequel l'agent de couplage est un composé époxy, un composé anhydride, ou un composé chlorure de diacyle.

6. Procédé de préparation d'un vaisseau sanguin artificiel biologique selon la revendication 2, dans lequel l'agent de couplage est un composé acétyl diamide, un composé anhydride dioïque, un composé diépoxy, ou tout autre composé ayant des groupes fonctionnels doubles, qui prend part à une réaction de condensation avec des groupes, tels que -NH₂, -OH, ou -COOH.

7. Procédé de préparation d'un vaisseau sanguin artificiel biologique selon la revendication 1, dans lequel le substrat 1 a été réticulé, et les antigènes sur le substrat 1 ont été minimisés, et le substrat 1 a une surface interne à laquelle un anticoagulant est couplé pour former le revêtement 2.

8. Procédé de préparation d'un vaisseau sanguin artificiel biologique selon la revendication 2, dans lequel lesdits composants du revêtement 2 sont des substances qui constituent les charges négatives de la charge de revêtement, ou des héparines ayant une grande activité anticoagulante.

9. Procédé de préparation d'un vaisseau sanguin artificiel biologique selon la revendication 2, dans lequel l'un desdits polypeptides est constitué de 16 lysines (K16), glycine (G), arginine (R), acide aspartique (D), sérine (S), proline(P) et cystéine (C).

10. Procédé de préparation d'un vaisseau sanguin artificiel biologique selon la revendication 9, dans lequel ledit substrat 1 a la forme d'un tube droit, d'un tube en forme de U, ou d'un tube en forme de C, ou d'un tube en forme de Y.
